(19) Europäisches Patentamt
European Patent Office
Office européen des brevets

(11) EP 2 349 394 B1

(12) EUROPÄISCHE PATENTSCHRIFT

(45) Veröffentlichungstag und Bekanntmachung des Hinweises auf die Patenterteilung:
04.03.2015 Patentblatt 2015/10

(21) Anmeldenummer: 09765018.8

(22) Anmeldetag: 14.11.2009

(51) Int Cl.:
A61M 1/36 (2006.01)

(86) Internationale Anmeldenummer:
PCT/EP2009/008128

(87) Internationale Veröffentlichungsnummer:
WO 2010/057600 (27.05.2010 Gazette 2010/21)

(54) **VERFAHREN UND VORRICHTUNG ZUR ERKENNUNG EINER PARAVASALEN BLUTUNG**

METHOD AND DEVICE FOR RECOGNITION OF PARAVASAL BLEEDING

PROCÉDÉ ET DISPOSITIF POUR DÉTECTER UNE HÉMORRAGIE PARAVASCULAIRE

(84) Benannte Vertragsstaaten:
AT BE BG CH CY CZ DE DK EE ES FI FR GB GR HR HU IE IS IT LI LT LU LV MC MK MT NL NO PL PT RO SE SI SK SM TR

(30) Priorität: 19.11.2008 DE 102008057994

(43) Veröffentlichungstag der Anmeldung:
03.08.2011 Patentblatt 2011/31

(73) Patentinhaber: Fresenius Medical Care Deutschland GmbH
61352 Bad Homburg v.d.H. (DE)

(72) Erfinder:
• KOPPERSCHMIDT, Pascal
97456 Dittelbrunn (DE)
• GROSS, Malte
89081 Ulm (DE)
• SPICKERMANN, Reiner
97535 Wasserlosen-Burghausen (DE)

(74) Vertreter: Oppermann, Frank et al
OANDO Oppermann & Oppermann LLP
Washingtonstrasse 75
65189 Wiesbaden (DE)

(56) Entgegenhaltungen:
EP-A1- 1 399 204     EP-A1- 1 930 035
EP-A2- 0 995 451     WO-A1-2005/105182
DE-A1- 10 254 988    DE-A1-102006 032 815
US-A- 4 846 792

**Beschreibung**

Die Erfindung bezieht sich auf ein Verfahren und eine Vorrichtung zur Erkennung einer paravasalen Blutung bei einer Zufuhr von Blut zu einem Gefäßzugang über eine Leitung und/oder der Entnahme von Blut aus einem Gefäßzugang über eine Leitung, insbesondere einer paravasalen Blutung während einer extrakorporalen Blutbehandlung, beispielsweise zur Hämodialyse, Hämofiltration oder Hämodiafiltration. Darüber hinaus betrifft die Erfindung eine Vorrichtung zur extrakorporalen Blutbehandlung, insbesondere zur Hämodialyse, Hämofiltration oder Hämodiafiltration, die über eine Vorrichtung zur Erkennung einer paravasalen Blutung verfügt.

Vorrichtungen für die Zufuhr von Blut zu einem Gefäßzugang und/oder der Entnahme von Blut aus einem Gefäßzugang über eine Leitung sind allgemein bekannt. Diese Vorrichtungen zeichnen sich dadurch aus, dass das Blut über eine Leitung dem Gefäßzugang, beispielsweise einer Vene oder Arterie zugeführt bzw. von dem Gefäßzugang abgeführt wird. Derartige Vorrichtungen sind beispielsweise als Infusions- oder Transfusionsvorrichtungen bekannt.

Auch bei den bekannten Vorrichtungen zur extrakorporalen Blutbehandlung, beispielsweise zur Hämodialyse, Hämofiltration oder Hämodiafiltration, handelt es sich um Vorrichtungen, bei denen über eine Leitung Blut einem Gefäßzugang zugeführt und Blut einem Gefäßzugang über eine Leitung entnommen wird.

Bei den bekannten Verfahren zur Blutbehandlung, beispielsweise der Hämodialyse, Hämofiltration oder Hämodiafiltration, wird das Blut des Patienten über einen extrakorporalen Blutkreislauf geleitet, der die Einrichtung zur Blutbehandlung enthält. Der extrakorporale Blutkreislauf ist im Allgemeinen mit einem Schutzsystem ausgestattet, das permanent den arteriellen und venösen Druck innerhalb des Kreislaufs überwacht. Zweck der Drucküberwachung ist die Erkennung von verschiedenen Komplikationen, die während der extrakorporalen Blutbehandlung auftreten können. Zu den möglichen Komplikationen zählt ein fehlerhafter Gefäßzugang, der beispielsweise auf das Herausrutschen oder Ansaugen der Kanüle zurück zu führen ist.

Die bekannten Schutzsysteme sehen bei der Feststellung eines fehlerhaften Gefäßzugangs in der Regel vor, die Blutpumpe zum Fördern des Bluts im extrakorporalen Blutkreislauf zu stoppen, eine Schlauchklemme in der venösen Blutleitung des extrakorporalen Kreislaufs zu schließen sowie ein akustisches oder optisches Warnsignal zu geben. Hierdurch wird die Blutbehandlungsvorrichtung in einen für den Patienten sicheren Zustand gebracht, der allerdings zur Unterbrechung der Therapie führt.

Verfahren und Vorrichtungen zur Überwachung eines extrakorporalen Blutkreislaufs, die auf der Überwachung des Drucks sowohl im venösen als auch arteriellen Zweig des extrakorporalen Kreislaufs beruhen, sind beispielsweise aus der DE 10 2006 032 815 A1 und EP 0 995 451 A2 bekannt.

Eine weitere Komplikation, die beispielsweise bei einer extrakorporalen Blutbehandlung auftreten kann, ist eine Verletzung des Gefäßzugangs, die zu einem Einbluten in das umgebende Gewebe führt, was auch als paravasale Blutung bezeichnet wird. Da paravasale Blutungen nur zu einem sehr langsamen Druckanstieg in der zu der Punktionsnadel führenden Leitung oder der Punktionsnadel selbst führen, ist die Erkennung paravasaler Blutungen in der Praxis problematisch. Wenn der Druck in der Leitung oder Kanüle noch weit unterhalb des Druckgrenzwertes liegt, bei dem die bekannten Drucküberwachungsvorrichtungen ansprechen, wird der nur geringe Druckanstieg pro Zeiteinheit zunächst nicht bemerkt, obwohl dabei kontinuierlich immer mehr Blut in das Gewebe einblutet und sich dabei akkumuliert, wodurch der Patient immer mehr geschädigt wird. Daher vergeht bis zum Erreichen des Druckgrenzwertes ein beträchtlicher Zeitraum, in dem Blut unerkannt in das Gewebe einblutet.

Weitere Verfahren und Vorrichtungen zur Erkennung eines nicht ordnungsgemäßen Gefäßzugangs, die auf einer Überwachung von Drucksignalen beruhen, sind beispielsweise aus der US-A-4,530,696, US-A-5,423,743 oder US-A-4,846,792 bekannt.

Aus der EP 0 817 653 B1 ist eine Vorrichtung bekannt, die auf eine Punktionskanüle aufgesetzt wird, um den Eintritt von Blut in die Kanüle zu erkennen. Der Bluteintritt beim Anstechen des Blutgefäßes wird dadurch detektiert, dass mittels eines Drucksensors ein Druckanstieg gemessen wird. Mit der bekannten Vorrichtung kann auch das Durchstechen der gegenüberliegenden Gefäßwand über den Druckabfall erkannt werden, der beim Durchstechen der gegenüberliegenden Gefäßwand eintritt.

Die EP 1 930 035 A1 beschreibt eine Vorrichtung, bei der ein Patientenzugang (vascular access) eines extrakorporalen Blutkreislaufs überwacht wird. Der Überwachung des Patientenzugangs liegt ein mathematisches Modell zugrunde. Die bekannte Überwachungsvorrichtung sieht die Messung sowohl des arteriellen als auch venösen Drucks im extrakorporalen Kreislauf vor. Das mathematische Modell beschreibt die Veränderung des Drucks am Patientenzugang (Fistel) als Funktion der Blutflussrate. Bei der weiteren Berechnung der zu bestimmenden Größen, bspw. der Blutflussrate stromauf des Patientenzugangs und des hydraulischen Widerstands zwischen dem arteriellen und venösen Zugang findet eine nicht-lineare Funktion Verwendung, bspw. ein Polynom.

Der Erfindung liegt die Aufgabe zugrunde, ein Verfahren anzugeben, das bei einer Zufuhr von Blut zu einem Gefäßzugang über eine Leitung und/oder der Entnahme von Blut aus einem Gefäßzugang über eine Leitung, insbesondere bei einer extrakorporalen Blutbehandlung, eine schnelle Erkennung einer paravasalen Blutung mit einer relativ hohen Sicherheit ermöglicht. Eine weitere Aufgabe der Erfindung ist, eine Vorrichtung zur Erkennung einer paravasalen Blutung zu schaffen, die bei einer Zufuhr von Blut zu einem Gefäßzugang und/oder der Entnahme von Blut aus einem Gefäßzugang, insbesondere bei einer extrakorporalen Blutbehandlung, eine schnelle Erkennung einer paravasalen Blutung mit einer relativ hohen Sicherheit erlaubt. Darüber hinaus ist eine Aufgabe der Erfindung eine extrakorporale Blutbehandlungsvorrichtung mit einer Vorrichtung zur Erkennung einer paravasalen Blutung anzugeben, die mit relativ hoher Sicherheit eine paravasale Blutung schnell erkennt.

Die Lösung dieser Aufgaben erfolgt erfindungsgemäß mit den Merkmalen der Patentansprüche 1, 10 und 20. Vorteilhafte Ausführungsformen der Erfindung sind Gegenstand der Unteransprüche.

Das Grundprinzip der Erfindung liegt darin, dass fortlaufende Druckzunahmen, die bei einem Druckniveau beginnen, das weit unterhalb des bei den bekannten Überwachungsvorrichtungen festgelegten Druckgrenzwerts liegt, mit Hilfe eines Auswertalgorithmus zur dynamischen Druckanalyse überproportional Berücksichtigung finden, weil bei solchen Ausgangszuständen die Gefahr einer besonders lang andauernden paravasalen Blutung bis zu deren Erkennung mittels der bekannten Überwachungsvorrichtungen besteht.

Da Druckänderungen fernab des vorgegebenen Referenzwerts einen größeren Beitrag zur Auswertung leisten sollen, wird erfindungsgemäß der Wert einer geeigneten nichtlinearen Funktion der Differenz zwischen dem gemessenen Druck und einem vorgegebenen Referenzwert bestimmt. Grundsätzlich kann jede nichtlineare Funktion verwandt werden, die für große Beträge der Differenz zwischen dem gemessenen Druck und einem vorgegebenen Referenzwert größere Beiträge liefert als für kleine Beträge der Differenz zwischen dem gemessenen Druck und einem vorgegebenen Referenzwert. Es kann beispielsweise eine Potenzfunktion verwandt werden, wobei beispielsweise die Differenz zwischen dem gemessenen Druck und einem vorgegebenen Referenzwert potenziert wird. Insbesondere kann bevorzugt der Betrag aus der Differenz zwischen dem gemessenen Druck und einem vorgegebenen Referenzwert potenziert werden. In der Praxis hat sich gezeigt, dass der Betrag aus der Differenz zwischen dem gemessenen Druck und einem vorgegebenen Referenzwert mit einer Potenz aus dem Wertebereich von 2 bis 4 belegt sein sollte. Dabei muss der Exponent nicht 2, 3 oder 4 sein, sondern der Exponent kann grundsätzlich auch jeder beliebige Zwischenwert in dem angegebenen Wertebereich von 2 bis 4 sein. Als besonders vorteilhaft hat sich in Laborversuchen die dreifache Potenz erwiesen. Je größer der Wert der Potenz ist, desto größer sind die Beiträge von Druckänderungen fernab des Referenzwertes.

Für den Fall, dass der Wert der nichtlinearen Funktion einen vorgegebenen Grenzwert überschreitet, wird auf das Vorliegen einer paravasalen Blutung geschlossen.

Bei der extrakorporalen Blutbehandlung beruhen das erfindungsgemäße Verfahren und die erfindungsgemäße Vorrichtung darauf, dass die Änderung des arteriellen Drucks im arteriellen Zweig und/oder des venösen Drucks im venösen Zweig erfasst wird. Wenn während der Blutbehandlung eine paravasale Blutung auftritt, zeigt sich, dass der positive Staudruck an der zu dem Gefäßzugang führenden Leitung, d.h. an der venösen Kanüle ansteigt, oder der Saugdruck an der von dem Gefäßzugang abgehenden Leitung, d.h. an der arteriellen Kanüle ansteigt, d.h. der Saugdruck negativer wird. Der Druckanstieg beginnt zunächst moderat und wird daraufhin zunehmend größer. Für das erfindungsgemäße Verfahren und die erfindungsgemäße Vorrichtung ist deshalb entscheidend, dass Druckänderungen, die von einem Druckniveau ausgehen, das einen großen Abstand vom Referenzwert aufweist, stärkere Berücksichtigung finden als solche, die von einem Druckniveau ausgehen, das nur einen geringen Abstand vom Referenzwert aufweist.

Das erfindungsgemäße Verfahren und die erfindungsgemäßen Vorrichtung können nicht nur den Druck an der venösen Nadel einer extrakorporalen Blutbehandlungsvorrichtung, sondern entsprechend auch den Druck an der arteriellen Nadel der extrakorporalen Blutbehandlungsvorrichtung überwachen, um beispielsweise eine an der Gefäßwand des Blutgefäßes angesaugte Punktionskanüle erkennen zu können.

Das erfindungsgemäße Verfahren und die erfindungsgemäße Vorrichtung können von den arteriellen und venösen Drucksensoren Gebrauch machen, die in den bekannten Blutbehandlungsvorrichtungen ohnehin vorhanden sind. Somit beschränkt sich die maschinenseitige Änderung zur Implementierung des Schutzsystems lediglich auf eine Modifikation der Maschinensteuerung.

Bei einer bevorzugten Ausführungsform der Erfindung wird das Integral des Wertes der Potenz des Betrags der Differenz zwischen dem gemessenen Druck und dem vorgegebenen Referenzwert über ein vorgegebenes Zeitintervall $t_2 - t_1$ bestimmt und mit dem vorgegeben arteriellen oder venösen Grenzwert verglichen, nach dessen Überschreiten auf das Vorliegen einer paravasalen Blutung geschlossen wird. Die Differenzen werden also kumuliert, bis der Wert des Integrals einen kritischen Wert überschreitet.

Das erfindungsgemäße Verfahren und die erfindungsgemäße Vorrichtung können auch mit anderen Verfahren und Vorrichtungen zur Erkennung eines fehlerhaften Gefäßzugangs einer extrakorporalen Blutbehandlung kombiniert werden. Damit kann die Sicherheit des Überwachungssystems noch weiter erhöht werden. Beispielsweise können das erfindungsgemäße Verfahren und die erfindungsgemäße Vorrichtung mit den Verfahren und Vorrichtungen kombiniert werden, die in der DE 10 2006 032 815 A1 und EP 0 995 451 A2 offenbart sind.

Durch die erfindungsgemäße Differenzüberwachung soll die konventionelle Überwachung des arteriellen bzw. venösen Drucks bei den bekannten Blutbehandlungsvorrichtungen nicht aufgehoben werden, sondern beide Überwachungssysteme können unabhängig voneinander arbeiten.

Bei fortwährenden Druckzunahmen aufgrund einer paravasalen Blutung ausgehend von einem Druckniveau nahe unterhalb des oberen Druckgrenzwerts der konventionellen Drucküberwachung wird bereits nach kurzer Zeit die konventionelle Drucküberwachung auf die Überschreitung des bekannten oberen Druckgrenzwerts reagieren. Eine überproportionale Berücksichtigung von Druckzunahmen ausgehend von einem solchen Druckniveau ist deshalb nicht erforderlich, weil nicht die Gefahr besteht, dass solche Druckzunahmen lange unbemerkt bleiben. Bei fortwährenden Druckzunahmen aufgrund einer paravasalen Blutung ausgehend von einem Druckniveau weit unterhalb des oberen Druckgrenzwerts der konventionellen Drucküberwachung werden erfindungsgemäß überproportional größere Beiträge zum Integral berechnet, so dass erfindungsgemäß frühzeitig auf eine paravasale Blutung geschlossen werden kann.

Für den Fall, dass eine paravasale Blutung auftritt, wird vorzugsweise ein akustischer und/oder optischer Alarm gegeben. Darüber hinaus kann der Blutfluss im extrakorporalen Blutkreislauf unterbrochen werden, um einen Blutverlust zu vermeiden. Eine Unterbrechung des Blutflusses ist bei den bekannten Vorrichtungen zur extrakorporalen Blutbehandlung dadurch möglich, dass die im extrakorporalen Blutkreislauf angeordnete Blutpumpe angehalten und/oder ein im extrakorporalen Kreislauf angeordnetes Sicherheitsventil, beispielsweise eine Schlauchklemme, insbesondere, die in der venösen Blutleitung angeordnete venöse Schlauchklemme geschlossen wird.

Das erfindungsgemäße Verfahren und die erfindungsgemäße Vorrichtung ist grundsätzlich zur Erkennung der unbeabsichtigten, unfallmäßigen Infusion von Blut in das ein Blutgefäß umgebende Gewebe eines Patienten infolge des unfallmäßigen Durchstoßens der gegenüberliegenden Gefäßwand des Blutgefäßes geeignet. Insbesondere ist das erfindungsgemäße Verfahren und die erfindungsgemäße Vorrichtung zur Anwendung bei allen Verfahren zur extrakorporalen Blutbehandlung geeignet, bei denen Blut über eine Kanüle in ein Blutgefäß des Patienten zurückgeführt wird. Beispiele dafür sind die Hämodialyse, Hämofiltration, Hämodiafiltration und auch Zellseparationsverfahren, bei denen das Blut eines Patienten in einem extrakorporalen Blutkreislauf einer Zellseparation unterworfen und dabei in seine Bestandteile getrennt wird.

Im Folgenden wird die Erfindung am Beispiel einer extrakorporalen Blutbehandlung unter Bezugnahme auf die Zeichnungen näher erläutert. Anhand eines Ausführungsbeispiels wird das erfindungsgemäße Verfahren und die erfindungsgemäße Vorrichtung zur Erkennung einer paravasalen Blutung bei einer extrakorporalen Blutbehandlung sowie die erfindungsgemäße extrakorporale Blutbehandlungsvorrichtung mit der Vorrichtung zur Erkennung einer paravasalen Blutung im Einzelnen beschrieben. Die Erfindung kann aber grundsätzlich auch bei allen anderen Zugängen zum Gefäßsystem eines Patienten Anwendung finden.

Es zeigen:

Fig. 1 in stark vereinfachter schematischer Darstellung eine Vorrichtung zur extrakorporalen Blutbehandlung, die über eine Vorrichtung zur Erkennung einer paravasalen Blutung verfügt und

Fig. 2 den Anstieg des venösen Drucks im venösen Zweig des extrakorporalen Blutkreislaufs beim Auftreten einer paravasalen Blutung.

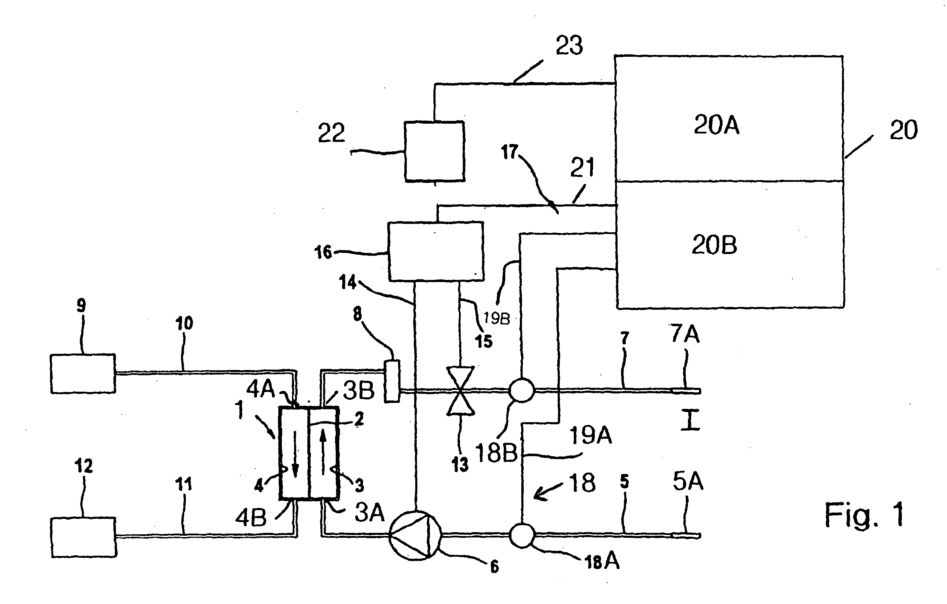

Fig. 1 zeigt in stark vereinfachter schematischer Darstellung eine Dialysevorrichtung als ein Ausführungsbeispiel einer extrakorporalen Blutbehandlungsvorrichtung.

Die Dialysevorrichtung weist als Blutbehandlungseinrichtung einen Dialysator 1 auf, der durch eine semipermeable Membran 2 in eine Blutkammer 3 und eine Dialysierflüssigkeitskammer 4 unterteilt ist. An dem Einlass 3A der Blutkammer 3 ist eine arterielle Blutleitung 5 angeschlossen, in die eine peristaltische Blutpumpe 6 geschaltet ist. Von dem Auslass 3B der Blutkammer 3 führt eine venöse Blutleitung 7 zu dem Patienten. In die venöse Blutleitung 7 ist eine Tropfkammer 8 geschaltet. An den Enden der arteriellen und venösen Blutleitung 5, 7 sind Kanülen 5A und 7A angeschlossen, die jeweils in ein entsprechendes arterielles bzw. venöses Blutgefäß (Shunt) des Patienten gestochen werden. Die arterielle und venöse Blutleitung 5, 7 bilden den arteriellen bzw. venösen Zweig des extrakorporalen Blutkreislaufs I. Die Blutleitungen sind Bestandteil eines als Disposable ausgebildeten Schlauchleitungssystems, das in die Dialysevorrichtung eingelegt wird.

In einer Dialysierflüssigkeitsquelle 9 wird frische Dialysierflüssigkeit bereitgestellt. Von der Dialysierflüssigkeitsquelle 9 führt eine Dialysierflüssigkeitszuführleitung 10 zu dem Eingang 4A der Dialysierflüssigkeitskammer 4A des Dialysators 1, während eine Dialysierflüssigkeitsabführleitung 11 von dem Ausgang 4B der Dialysierflüssigkeitskammer 4 zu einem Abfluss 12 führt.

Die Dialysevorrichtung kann noch über weitere Komponenten, beispielsweise eine Bilanziereinrichtung und eine Ultrafiltrationseinrichtung etc. verfügen, die der besseren Übersichtlichkeit halber aber nicht dargestellt sind.

Darüber hinaus verfügt die Dialysevorrichtung über Mittel zur Unterbrechung des Blutflusses im Falle eines fehlerhaften Gefäßzugangs. Zur Unterbrechung des Blutflusses ist an der venösen Blutleitung 7 stromab der Tropfkammer 8 eine Absperrklemme 13 vorgesehen, die elektromagnetisch betätigt wird. Die arterielle Blutpumpe 6 und die venöse Absperrklemme 13 werden über Steuerleitungen 14, 15 von einer zentralen Steuereinheit 16 der Dialysevorrichtung angesteuert.

Die Überwachung des extrakorporalen Blutkreislaufs zur Erkennung einer paravasalen Blutung kann bei den bekannten Blutbehandlungsvorrichtungen, die im sogenannten Zwei-Nadelbetrieb mit einer arteriellen und venösen Kanüle zur Herstellung des Patientenzugangs arbeiten, und bei den bekannten Dialysemaschinen im Einnadel-Betrieb mit nur einer Kanüle für den arteriellen und venösen Patientenzugang erfolgen.

Die Vorrichtung 17 zur Erkennung einer paravasalen Blutung verfügt über eine Einrichtung 18 zur Messung des arteriellen Drucks im arteriellen Zweig 5 und des venösen Drucks im venösen Zweig 7 des extrakorporalen Blutkreislaufs I. Die Messeinrichtung 18 weist einen den Druck in der arteriellen Blutleitung 5 überwachenden arteriellen Drucksensor 18A und einen den Druck in der venösen Blutleitung 7 überwachenden venösen Drucksensor 18B auf. Die Messwerte der Drucksensoren 18A, 18B werden über Datenleitungen 19A, 19B an eine Rechen- und Auswerteinheit 20 der Vorrichtung 17 zur Erkennung der paravasalen Blutung übertragen.

Die Auswert- und Recheneinheit 20 verfügt über Mittel zum Berechnen einer nichtlinearen Funktion der Differenz zwischen dem momentan gemessenen arteriellen bzw. venösen Druck $p(t)$ und einem vorgegebenen Referenzwert $p_{ref}$ nach der folgenden Gleichung:

$$I = f\left(p(t) - P_{ref}\right)$$

Gleichung (1)

Bei dem nachfolgenden Ausführungsbeispiel wird die nichtlineare Funktion wie folgt angenommen. Die Auswert- und Recheneinheit 20 verfügt über Mittel 20A zum Berechnen des Integrals I des Wertes der n-fachen Potenz des Betrags der Differenz zwischen dem momentan gemessenen arteriellen bzw. venösen Druck $p(t)$ und einem vorgegebenen Referenzwert $p_{ref}$ für den Druck für ein vorgegebenes Zeitintervall $t_2 - t_1$ nach der folgenden Gleichung:

$$I = \frac{1}{t_2 - t_1} \int_{t_1}^{t_2} \left|p(t) - p_{ref}\right|^n dt$$

Gleichung (2)

Als Referenzwert $p_{ref}$ *können* die nachfolgend genannten Werte angenommen werden.

1. $p_{ref}$ ist oberer Grenzdruck des Grenzwertfensters der bekannten Drucküberwachung
2. $p_{ref}$ ist unterer Grenzdruck des Grenzwertfensters der bekannten Drucküberwachung
3. $p_{ref}$ ist mittlerer (symmetrischer oder asymmetrischer) Grenzdruck des Grenzwertfensters der bekannten Drucküberwachung
4. $p_{ref}$ ist der mit einem Tiefpass gefilterte Wert des Drucks
5. $p_{ref}$ ist ein gleitend-gemittelter Wert des Drucks
6. $p_{ref}$ ist eine Konstante

Der Referenzwert kann auf verschiedene Weise vorgegeben werden. Der Referenzwert $p_{ref}$ und/oder der Exponent n und/oder das Zeitintervall $t_2 - t_1$ können in der Vorrichtung vom Benutzer manuell eingegeben und vorgegeben werden oder können in der Vorrichtung fest hinterlegt sein oder können von der Vorrichtung nach vorgegebenen Regeln automatisch berechnet und vorgeschlagen werden.

Weiterhin weist die Auswert- und Recheneinheit 20 Mittel 20B zum Vergleichen des Integrals I mit einem vorgegeben Grenzwert crit auf.

$$I \geq crit$$

Gleichung (3)

Das Integral I wird entsprechend Gleichung (2) sowohl für den mit dem arteriellen Drucksensor 18A gemessenen

arteriellen Druck $p_{art}(t)$ als auch für den mit dem Drucksensor 18B gemessenen venösen Druck $p_{ven}(t)$ berechnet, wobei das Integral $I_{art}$ mit dem vorgegebenen arteriellen Grenzwert $crit_{art}$ und das Integral $I_{ven}$ mit den vorgegebenen venösen Grenzwert $crit_{ven}$ verglichen wird.

Bei Überschreiten des Grenzwertes wird auf das Vorliegen einer paravasalen Blutung geschlossen.

Der Exponent n = 3 hat sich in Laborversuchen für die Potenzfunktion in der Gleichung (2) als besonders günstig erwiesen. Je größer der Wert des Index n gewählt wird, desto größer sind die Beiträge p(t) fernab von $p_{ref}$. Der Exponent kann aber grundsätzlich auch jeder beliebige Zwischenwert in dem Intervall von 2 bis 4 sein.

Wenn eine paravasale Blutung durch Überschreitung des Grenzwertes erkannt wird, erzeugt die Auswert- und Recheneinheit 20 ein Steuersignal, das die zentrale Steuereinheit 16 der Dialysevorrichtung über eine Steuerleitung 21 empfängt. Daraufhin stoppt die Steuereinheit 16 die Blutpumpe 6 und schließt die venöse Absperrklemme 13, so dass der Blutfluss im extrakorporalen Blutkreislauf I unterbrochen ist. Folglich kann kein Blut mehr in das Peripheriegewebe einbluten.

Die Rechen- und Auswerteinheit 20 weist weiterhin eine Alarmeinheit 22 auf, die über einen Datenleitung 23 mit der zentralen Steuereinheit 16 der Dialysevorrichtung verbunden ist. Die Alarmeinheit 22 gibt einen akustischen und/oder optischen Alarm, wenn eine paravasale Blutung erkannt wird.

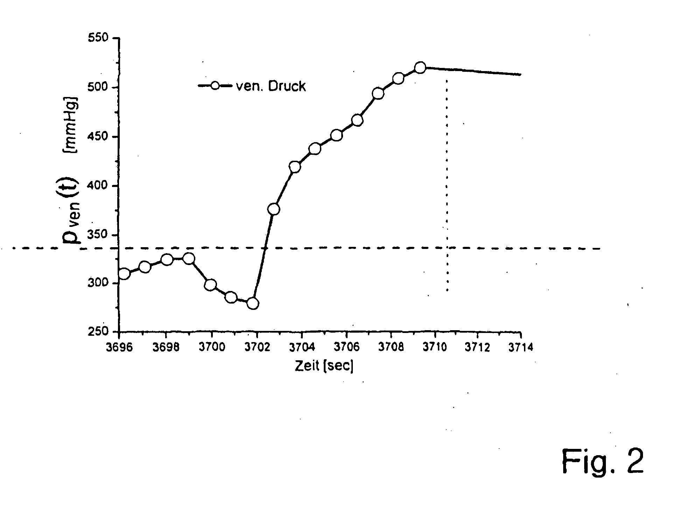

Fig. 2 zeigt für den Fall einer paravasalen Blutung an der venösen Kanüle 7A den Verlauf des mit dem venösen Drucksensor 18B gemessenen venösen Drucks $p_{ven}(t)$ in der venösen Blutleitung 7 stromauf der venösen Kanüle 7A vor und nach dem Auftreten der paravasalen Blutung bei einer konventionellen Drucküberwachung mit einem bekannten Schutzsystem nach dem Stand der Technik. Es zeigt sich ein Druckanstieg beim Auftreten der paravasalen Blutung, wobei der venöse Druck zunächst langsam und dann deutlich schneller ansteigt. Die obere Grenze für den venösen Druck, die mit dem konventionellen Überwachungssystem überwacht wird, ist in Fig. 2 in gestrichelten Linien eingezeichnet. Diese liegt bei dem vorliegenden Ausführungsbeispiel bei ca. 330 mmHg. Die Blutbehandlung wird bei dem konventionellen Überwachungssystem grundsätzlich nach Erreichen oder Überschreiten der oberen Grenze für den venösen Druck durch Stoppen der Blutpumpe 6 und Schließen der Schlauchklemme 13 unterbrochen.

Aus Fig. 2 ist aber ersichtlich, dass die Blutpumpe erst bei ca. t = 3711 s gestoppt wird, obwohl der bekannte obere Grenzwert, der in Fig. 2 durch die gestrichelte waagerechte Linie dargestellt ist, bereits seit ca. 9 s überschritten ist. Der Grund dafür ist, dass das konventionelle Schutzsystem in der Dialysemaschine eine Reaktionsverzögerung in Form eines fest vorgegebenen Alarmfensters vorsieht, so dass die Unterbrechung des Blutflusses erst nach einer vorgegebenen Zeitverzögerung erfolgt. In diesem Zeitraum kann bei üblichen Blutflüssen ca. 120 ml Blut gefördert werden, d.h. es werden bei Erkennung einer paravasalen Blutung durch Überschreitung des bekannten Grenzwerts noch weitere 120 ml Blut in das Gewebe des Patienten gepumpt. Diese Reaktionsverzögerung hat die Aufgabe, bei den häufigeren Drucküberschreitungen, die meist nur kurzfristig auftreten und meist auf harmlose Ursachen zurückzuführen sind, die Rate der Fehlalarme zu senken, d.h. es soll nicht stets sofort mit dem Abschalten der Pumpe reagiert werden. Insofern ergibt sich bei dem konventionellen Schutzsystem ein Zielkonflikt zwischen dem Wunsch der frühzeitigen Erkennung einer in der Praxis relativ seltenen, aber folgenschweren paravasalen Blutung und der Minimierung von Fehlalarmen. Deshalb geht die Erfindung davon aus, dass das bekannte Schutzsystem vorzugsweise bestehen bleibt und das erfindungsgemäße Schutzsystem dazu parallel arbeitet, wodurch die Sicherheit der Überwachung erhöht wird und Fehlalarme weitgehend vermieden werden.

## Patentansprüche

1. Verfahren zur Erkennung einer paravasalen Blutung bei einer Zufuhr von Blut zu einem Gefäßzugang über eine Leitung und/oder der Entnahme von Blut aus einem Gefäßzugang über eine Leitung, wobei der Druck in der Leitung gemessen wird, **dadurch gekennzeichnet, dass** mit einer Auswert- und Recheneinheit eine nichtlineare Funktion der Differenz zwischen dem in der Leitung gemessenen Druck und einem vorgegebenen Referenzwert bestimmt wird, wobei mit der Auswert- und Recheneinheit der Wert der nichtlinearen Funktion mit einem vorgegebenen Grenzwert verglichen wird und bei Überschreiten des Grenzwertes auf das Vorliegen einer paravasalen Blutung geschlossen wird.

2. Verfahren nach Anspruch 1, **dadurch gekennzeichnet, dass** der Wert der Potenz der Differenz zwischen dem gemessenen Druck und dem vorgegebenen Referenzwert bestimmt wird, wobei der Wert der Potenz der Druckdifferenz mit dem vorgegebenen Grenzwert verglichen wird und bei Überschreiten des Grenzwertes auf das Vorliegen einer paravasalen Blutung geschlossen wird.

3. Verfahren nach Anspruch 1 oder 2, **dadurch gekennzeichnet, dass** der Wert der Potenz des Betrags der Differenz zwischen dem gemessenen Druck und dem vorgegebenen Referenzwert bestimmt wird, wobei der Wert der Potenz des Betrags der Druckdifferenz mit dem vorgegebenen Grenzwert verglichen wird und bei Überschreiten des Grenzwertes auf das Vorliegen einer paravasalen Blutung geschlossen wird.

4. Verfahren nach einem der Ansprüche 1 bis 3 zur Erkennung einer paravasalen Blutung während einer extrakorporalen Blutbehandlung, bei der Blut von einem arteriellen Gefäßzugang über einen arteriellen Zweig eines extrakorporalen Blutkreislaufs in eine Blutbehandlungseinrichtung strömt und aus der Blutbehandlungseinrichtung über einen venösen Zweig des extrakorporalen Kreislaufs zu einem venösen Gefäßzugang strömt, wobei der arterielle Druck im arteriellen Zweig und/oder der venöse Druck im venösen Zweig des extrakorporalen Kreislaufs gemessen wird,
**dadurch gekennzeichnet, dass**
ein erster Wert einer nichtlinearen Funktion der Differenz zwischen dem gemessenen arteriellen Druck und einem vorgegebenen Referenzwert für den arteriellen Druck und/oder
ein zweiter Wert einer nichtlinearen Funktion der Differenz zwischen dem gemessenen venösen Druck und einem vorgegebenen Referenzwert für den venösen Druck bestimmt wird, wobei der erste Wert mit einem ersten Grenzwert und/oder der zweite Wert mit einem zweiten Grenzwert verglichen wird und bei Überschreiten des ersten Grenzwerts bzw. des zweiten Grenzwerts auf das Vorliegen einer paravasalen Blutung geschlossen wird.

5. Verfahren nach Anspruch 4, **dadurch gekennzeichnet, dass** ein erster Wert der Potenz des Betrags der Differenz zwischen dem gemessenen arteriellen Druck und einem vorgegebenen Referenzwert für den arteriellen Druck und/oder ein zweiter Wert der Potenz des Betrags der Differenz zwischen dem gemessenen venösen Druck und einem vorgegebenen Referenzwert für den venösen Druck bestimmt wird, wobei der erste Wert mit einem ersten Grenzwert und/oder der zweite Wert mit einem zweiten Grenzwert verglichen wird und bei Überschreiten des ersten Grenzwerts bzw. des zweiten Grenzwerts auf das Vorliegen einer paravasalen Blutung geschlossen wird.

6. Verfahren nach Anspruch 4, **dadurch gekennzeichnet, dass** ein erster Wert des Integrals der Potenz des Betrags der Differenz zwischen dem gemessenen arteriellen Druck und einem vorgegebenen Referenzwert für den arteriellen Druck über ein vorgegebenes Intervall $t_2 - t_1$ bestimmt wird und der erste Wert mit einem ersten vorgegebenen Grenzwert verglichen wird und bei Überschreiten des ersten Grenzwerts auf das Vorliegen einer paravasalen Blutung geschlossen wird und/oder ein zweiter Wert des Integrals der Potenz des Betrags der Differenz zwischen dem gemessenen venösen Druck und einem vorgegebenen Referenzwert für den venösen Druck über ein vorgegebenes Intervall $t_2 - t_1$ bestimmt wird und der zweite Wert mit einem zweiten vorgegebenen Grenzwert verglichen wird und bei Überschreiten des zweiten Grenzwerts auf das Vorliegen einer paravasalen Blutung geschlossen wird.

7. Verfahren nach Anspruch 5 oder 6, **dadurch gekennzeichnet, dass** die zweifache, dreifache oder vierfache Potenz des Betrags der Differenz zwischen dem gemessenen arteriellen Druck und einem vorgegebenen Referenzwert für den arteriellen Druck bzw. die zweifache, dreifache oder vierfache Potenz des Betrags der Differenz zwischen dem gemessenen venösen Druck und einem vorgegebenen Referenzwert für den venösen Druck bestimmt wird

8. Verfahren nach einem der Ansprüche 1 bis 7, **dadurch gekennzeichnet, dass** bei Feststellung einer paravasalen Blutung ein optischer und/oder akustischer Alarm gegeben wird.

9. Verfahren nach einem der Ansprüche 1 bis 8, **dadurch gekennzeichnet, dass** bei Feststellung einer paravasalen Blutung der Blutfluss unterbrochen wird.

10. Vorrichtung zur Erkennung einer paravasalen Blutung bei einer Zufuhr von Blut zu einem Gefäßzugang über eine Leitung und/oder der Entnahme von Blut aus einem Gefäßzugang über eine Leitung, wobei die Vorrichtung zur Erkennung einer paravasalen Blutung eine Einrichtung zur Messung des Drucks in der Leitung aufweist, **dadurch gekennzeichnet, dass** die Vorrichtung zur Erkennung einer paravasalen Blutung eine Auswert- und Recheneinheit (20) aufweist, die derart ausgebildet ist, dass eine nichtlineare Funktion der Differenz zwischen dem in der Leitung gemessenen Druck und einem vorgegebenen Referenzwert bestimmt wird, der Wert der nichtlinearen Funktion mit einem vorgegebenen Grenzwert verglichen wird und bei Überschreiten des Grenzwertes auf das Vorliegen einer paravasalen Blutung geschlossen wird.

11. Vorrichtung nach Anspruch 10, **dadurch gekennzeichnet, dass** die Auswert- und Recheneinheit (20) derart ausgebildet ist, dass der Wert der Potenz der Differenz zwischen dem gemessenen Druck und dem vorgegebenen Referenzwert bestimmt wird, wobei der Wert der Potenz der Druckdifferenz mit dem vorgegebenen Grenzwert verglichen wird und bei Überschreiten des Grenzwertes auf das Vorliegen einer paravasalen Blutung geschlossen wird.

**12.** Vorrichtung nach Anspruch 10 oder 11, **dadurch gekennzeichnet, dass** die Auswert- und Recheneinheit (20) derart ausgebildet ist, dass der Wert der Potenz des Betrags der Differenz zwischen dem gemessenen Druck und dem vorgegebenen Referenzwert bestimmt wird, wobei der Wert der Potenz des Betrags der Druckdifferenz mit dem vorgegebenen Grenzwert verglichen wird und bei Überschreiten des Grenzwertes auf das Vorliegen einer paravasalen Blutung geschlossen wird.

**13.** Vorrichtung nach einem der Ansprüche 10 bis 12 zur Erkennung einer paravasalen Blutung während einer extrakorporalen Blutbehandlung mit einer extrakorporalen Blutbehandlungsvorrichtung, wobei die extrakorporale Blutbehandlungsvorrichtung aufweist:

eine arterielle Blutleitung eines extrakorpralen Blutkreislaufs, die an einem Ende mit dem Einlass einer Blutbehandlungseinrichtung verbunden ist und an dem anderen Ende mit einem arteriellen Anschluss für einen arteriellen Gefäßzugang versehen ist,
eine venöse Blutleitung des extrakorpralen Blutkreislaufs, die an einem Ende mit dem Auslass der Blutbehandlungseinrichtung verbunden ist und an dem anderen Ende mit einem venösen Anschluss für einen venösen Gefäßzugang versehen ist,
und eine Einrichtung zur Messung des arteriellen Drucks im arteriellen Zweig und/oder des venösen Drucks im venösen Zweig des extrakorporalen Blutkreislaufs,
**dadurch gekennzeichnet, dass**
die Vorrichtung zur Erkennung einer paravasalen Blutung eine Auswert- und Recheneinheit (20) aufweist, die derart ausgebildet ist, dass ein erster Wert einer nichtlinearen Funktion der Differenz zwischen dem gemessenen arteriellen Druck und einem vorgegebenen Referenzwert für den arteriellen Druck und/oder ein zweiter Wert einer nichtlinearen Funktion der Differenz zwischen dem gemessenen venösen Druck und einem vorgegebenen Referenzwert für den venösen Druck bestimmt wird, wobei der erste Wert mit einem ersten vorgegebenen Grenzwert und/oder der zweite Wert mit einem zweiten vorgegebenen Grenzwert verglichen wird und bei Überschreiten des ersten Grenzwerts bzw. des zweiten Grenzwerts auf das Vorliegen einer paravasalen Blutung geschlossen wird.

**14.** Vorrichtung nach Anspruch 13, **dadurch gekennzeichnet, dass** die Vorrichtung zur Erkennung einer paravasalen Blutung eine Auswert- und Recheneinheit (20) aufweist, die derart ausgebildet ist, dass ein erster Wert der Potenz des Betrags der Differenz zwischen dem gemessenen arteriellen Druck und einem vorgegebenen Referenzwert für den arteriellen Druck und/oder ein zweiter Wert der Potenz des Betrags der Differenz zwischen dem gemessenen venösen Druck und einem vorgegebenen Referenzwert für den venösen Druck bestimmt wird, wobei der erste Wert mit einem ersten vorgegebenen Grenzwert und/oder der zweite Wert mit einem zweiten vorgegebenen Grenzwert verglichen wird und bei Überschreiten des ersten Grenzwerts bzw. des zweiten Grenzwerts auf das Vorliegen einer paravasalen Blutung geschlossen wird.

**15.** Vorrichtung nach Anspruch 13, **dadurch gekennzeichnet, dass** dass die Vorrichtung zur Erkennung einer paravasalen Blutung eine Auswert- und Recheneinheit (20) aufweist, die aufweist:

Mittel (20A) zum Berechnen eines ersten Werts des Integrals der Potenz des Betrags der Differenz zwischen dem gemessenen arteriellen Druck und einem Referenzwert für den arteriellen Druck über ein vorgegebenes Zeitintervall $t_2$ - $t_1$ und/oder eines zweiten Werts des Integrals der Potenz des Betrags der Differenz zwischen dem gemessenen venösen Druck und einem Referenzwert für den venösen Druck über ein vorgegebenes Zeitintervall $t_2$ - $t_1$ und
Mittel (20B) zum Vergleichen des ersten Werts mit einem ersten vorgegebenen Grenzwert und/oder des zweiten Werts mit einem zweiten vorgegebenen Grenzwert.

**16.** Vorrichtung nach Anspruch 14 oder 15, **dadurch gekennzeichnet, dass** die Auswert- und Recheneinheit (20) derart ausgebildet ist, dass die zweifache, dreifache oder vierfache Potenz des Betrags der Differenz zwischen dem gemessenen arteriellen Druck und einem vorgegebenen Referenzwert für den arteriellen Druck bzw. die zweifache, dreifache oder vierfache Potenz des Betrags der Differenz zwischen dem gemessenen venösen Druck und einem vorgegebenen Referenzwert für den venösen Druck bestimmt wird.

**17.** Vorrichtung nach einem der Ansprüche 10 bis 16, **dadurch gekennzeichnet, dass** die Vorrichtung zur Erkennung einer paravasalen Blutung eine Alarmeinheit (22) aufweist, der bei Feststellung einer paravasalen Blutung einen optischen und/oder akustischen Alarm gibt.

18. Vorrichtung nach einem der Ansprüche 13 bis 17, **dadurch gekennzeichnet, dass** die Vorrichtung zur Erkennung einer paravasalen Blutung Mittel (20) zur Erzeugung eines Steuersignals für die Unterbrechung des Blutflusses bei der Feststellung einer paravasalen Blutung aufweist.

19. Vorrichtung nach einem der Ansprüche 13 bis 18, **dadurch gekennzeichnet, dass** die Vorrichtung zur Erkennung einer paravasalen Blutung Mittel (20) zur Erzeugung eines Steuersignals für die Unterbrechung des Blutflusses der extrakoporalen Blutbehandlungsvorrichtung bei der Feststellung einer paravaselen Blutung aufweist.

20. Extrakorporale Blutbehandlungsvorrichtung mit
einer arteriellen Blutleitung (5) eines extrakorpralen Blutkreislaufs (I), die an einem Ende mit dem Einlass (3A) einer Blutbehandlungseinrichtung (1) verbunden ist und an dem anderen Ende mit einem arteriellen Anschluss (5A) für einen arteriellen Gefäßzugang versehen ist,
einer venösen Blutleitung (7) des extrakorporalen Blutkreislaufs (I), die an einem Ende mit dem Auslass (3B) der Blutbehandlungseinrichtung (1) verbunden ist und an dem anderen Ende mit einem venösen Anschluss (7A) für einen venösen Gefäßzugang versehen ist,
und einer Einrichtung (18) zur Messung des arteriellen Drucks im arteriellen Zweig (5) und/oder des venösen Drucks im venösen Zweig (7) des extrakorporalen Blutkreislaufs (I),
**dadurch gekennzeichnet, dass** die extrakorporale Blutbehandlungsvorrichtung eine Vorrichtung nach einem der Ansprüche 13 bis 19 aufweist.

21. Extrakorporale Blutbehandlungsvorrichtung nach Anspruch 20, **dadurch gekennzeichnet, dass** die extrakorporale Blutbehandlungsvorrichtung Mittel (6, 13) zum Unterbrechen des Blutflusses im extrakorporalen Blutkreislauf aufweist.

## Claims

1. Method of detecting perivascular bleeding when blood is being fed to a vascular access via a line and/or blood is being taken from a vascular access via a line, the pressure in the line being measured, **characterised in that** a non-linear function of the difference between the pressure which is measured in the line and a preset reference value is determined by an analysing and calculating unit, the value of the non-linear function being compared with a preset limiting value by the analysing and calculating unit and it being concluded that there is perivascular bleeding if the limiting value is exceeded.

2. Method according to claim 1, **characterised in that** the value of a power of the difference between the pressure which is measured and the preset reference value is determined, the value of the power of the difference in pressure being compared with the preset limiting value and it being concluded that there is perivascular bleeding if the limiting value is exceeded.

3. Method according to claim 1 or 2, **characterised in that** the value of a power of the absolute value of the difference between the pressure which is measured and the preset reference value is determined, the value of the power of the absolute value of the difference in pressure being compared with the preset limiting value and it being concluded that there is perivascular bleeding if the limiting value is exceeded.

4. Method according to one of claims 1 to 3 of detecting perivascular bleeding during extra-corporeal blood treatment in which the blood flows from an arterial vascular access via an arterial segment of an extra-corporeal blood circuit into a blood treating means and from the blood treating means via a venous segment of the extra-corporeal blood circuit to a venous vascular access, the arterial pressure in the arterial segment of the extra-corporeal blood circuit and/or the venous pressure in its venous segment being measured, **characterised in that** a first value, of a non-linear function of the difference between the arterial pressure which is measured and a preset reference value for the arterial pressure, is determined and/or a second value, of a non-linear function of the difference between the venous pressure which is measured and a preset reference value for the venous pressure, is determined, the first value being compared with a first limiting value and/or the second value being compared with a second limiting value and it being concluded that there is perivascular bleeding if the first limiting value and the second limiting value are exceeded in the respective cases.

5. Method according to claim 4, **characterised in that** a first value, of a power of the absolute value of the difference between the arterial pressure which is measured and a preset reference value for the arterial pressure is determined and/or a second value, of a power of the absolute value of the difference between the venous pressure which is measured and a preset reference value for the venous pressure is determined, the first value being compared with a first limiting value and/or the second value being compared with a second limiting value and it being concluded that there is perivascular bleeding if the first limiting value and the second limiting value are exceeded in the respective cases.

6. Method according to claim 4, **characterised in that** a first value of the integral of a power of the absolute value of the difference between the arterial pressure which is measured and a preset reference value for the arterial pressure is determined over a preset interval $t_2 - t_1$ and the first value is compared with a first preset limiting value and it is concluded that there is perivascular bleeding if the first limiting value is exceeded, and/or a second value of the integral of a power of the absolute value of the difference between the venous pressure which is measured and a preset reference value for the venous pressure is determined over a preset interval $t_2 - t_i$ and the second value is compared with a second preset limiting value and it is concluded that there is perivascular bleeding if the second limiting value is exceeded.

7. Method according to claim 5 or 6, **characterised in that** the second, third or fourth power of the absolute value of the difference between the arterial pressure which is measured and a preset reference value for the arterial pressure is determined, or the second, third or fourth power of the absolute value of the difference between the venous pressure which is measured and a preset reference value for the venous pressure is determined.

8. Method according to one of claims 1 to 7, **characterised in that** a visual and/or audio alarm is given when there is found to be perivascular bleeding.

9. Method according to one of claims 1 to 8, **characterised in that** the flow of blood is interrupted when there is found to be perivascular bleeding.

10. Arrangement for detecting perivascular bleeding when blood is being fed to a vascular access via a line and/or blood is being taken from a vascular access via a line, the arrangement for detecting perivascular bleeding having a means of measuring the pressure in the line, **characterised in that** the arrangement for detecting perivascular bleeding has an analysing and calculating unit (20) which is so designed that a non-linear function of the difference between the pressure which is measured in the line and a preset reference value is determined, the value of the non-linear function is compared with a preset limiting value and it is concluded that there is perivascular bleeding if the limiting value is exceeded.

11. Arrangement according to claim 10, **characterised in that** the analysing and calculating unit (20) is so designed that the value of a power of the difference between the pressure which is measured and the preset reference value is determined, in which case the value of the power of the difference in pressure is compared with the preset limiting value and it is concluded that there is perivascular bleeding if the limiting value is exceeded.

12. Arrangement according to claim 10 or 11, **characterised in that** the analysing and calculating unit (20) is so designed that the value of a power of the absolute value of the difference between the pressure which is measured and the preset reference value is determined, in which case the value of the power of the absolute value of the difference in pressure is compared with the preset limiting value and it is concluded that there is perivascular bleeding if the limiting value is exceeded.

13. Arrangement according to one of claims 10 to 12 for detecting perivascular bleeding during extra-corporeal blood treatment by an extra-corporeal blood treating apparatus, the extra-corporeal blood treating apparatus having an arterial blood line belonging to an extra-corporeal blood circuit, which arterial blood line is connected to the inlet of a blood treating means at one end and is provided with an arterial connection for an arterial vascular access at the other end, having a venous blood line belonging to the extra-corporeal blood circuit, which venous blood line is connected to the outlet of the blood treating means at one end and is provided with a venous connection for a venous vascular access at the other end, and having a means for measuring the arterial pressure in the arterial segment of the extra-corporeal blood circuit and/or the venous pressure in its venous segment, **characterised in that** the arrangement for detecting perivascular bleeding has an analysing and calculating unit (20) which is so designed that a first value, of a non-linear function of the difference between the arterial pressure which is measured and a preset reference value for the arterial pressure and/or a second value, of a non-linear function of the difference between the venous pressure which is measured and a preset reference value for the venous pressure is determined, in which case the first value is compared with a first preset limiting value and/or the second value is compared with a second preset limiting value and it is concluded that there is perivascular bleeding if the first limiting value and the second limiting value are exceeded in the respective cases.

14. Arrangement according to claim 13, **characterised in that** that the arrangement for detecting perivascular bleeding has an analysing and calculating unit (20) which is so designed that a first value, of a power of the absolute value of the difference between the arterial pressure which is measured and a preset reference value for the arterial pressure is determined and/or a second value, of a power of the absolute value of the difference between the venous pressure which is measured and a preset reference value for the venous pressure is determined, in which case the first value is compared with a first preset limiting value and/or the second value is compared with a second preset limiting value and it is concluded that there is perivascular bleeding if the first limiting value and the second limiting value are exceeded in the respective cases.

15. Arrangement according to claim 13, **characterised in that** the arrangement for detecting perivascular bleeding has an analysing and calculating unit (20) which has: means (20A) for calculating a first value of the integral of a power of the absolute value of the difference between the arterial pressure which is measured and a reference value for the arterial pressure, over a preset interval of time $t_2 - t_1$, and/or a second value of the integral of a power of the absolute value of the difference between the venous pressure which is measured and a reference value for the venous pressure, over a preset interval of time $t_2 - t_1$, and means (20B) for comparing the first value with a first preset limiting value and/or the second value with a second preset limiting value.

16. Arrangement according to claim 14 or 15, **characterised in that** the analysing and calculating unit (20) is so designed that the second, third or fourth power of the absolute value of the difference between the arterial pressure which is measured and a preset reference value for the arterial pressure is determined or the second, third or fourth power of the absolute value of the difference between the venous pressure which is measured and a preset reference value for the venous pressure is determined.

17. Arrangement according to one of claims 10 to 16, **characterised in that** the arrangement for detecting perivascular bleeding has an alarm unit (22) which gives a visual and/or audio alarm when there is found to be perivascular bleeding.

18. Arrangement according to one of claims 13 to 17, **characterised in that** the arrangement for detecting perivascular bleeding has means (20) for generating a control signal for interrupting the flow of blood when there is found to be perivascular bleeding.

19. Arrangement according to one of claims 13 to 18, **characterised in that** the arrangement for detecting perivascular bleeding has means (20) for generating a control signal for interrupting the flow of blood in the extra-corporeal blood treating apparatus when there is found to be perivascular bleeding.

20. Extra-corporeal blood treating apparatus having an arterial blood line (5) belonging to an extra-corporeal blood circuit (I), which arterial blood line is connected to the inlet (3A) of a blood treating means (1) at one end and is provided with an arterial connection (5A) for an arterial vascular access at the other end, having a venous blood line (7) belonging to the extra-corporeal blood circuit (I), which venous blood line is connected to the outlet (3B) of the blood treating means (1) at one end and is provided with a venous connection (7A) for a venous vascular access at the other end, and having a means (18) for measuring the arterial pressure in the arterial segment (5) of the extra-corporeal blood circuit (I) and/or the venous pressure in its venous segment (7), **characterised in that** the extra-corporeal blood treating apparatus has an arrangement according to one of claims 13 to 19.

21. Extra-corporeal blood treating apparatus according to claim 20, **characterised in that** the extra-corporeal blood treating apparatus has means (6, 13) for interrupting the flow of blood in the extra-corporeal blood circuit.

**Revendications**

1. Procédé de détection d'une hémorragie paravasculaire lors d'un apport de sang vers un accès vasculaire par l'intermédiaire d'une conduite et/ou lors du prélèvement de sang dans un accès à un vaisseau par l'intermédiaire d'une conduite, la pression dans la conduite étant mesurée, **caractérisé en ce que**, avec une unité d'analyse et de calcul, une fonction non linéaire de la différence entre la pression mesurée dans la conduite et une valeur de référence prédéterminée est déterminée, l'unité d'analyse et de calcul permettant de comparer la valeur de la fonction non linéaire avec une valeur limite prédéterminée et, lors du dépassement de la valeur limite, la présence d'une hémorragie paravasculaire en est déduite.

2. Procédé selon la revendication 1, **caractérisé en ce que** la valeur de la puissance de la différence entre la pression mesurée et la valeur de référence prédéterminée est déterminée, la valeur de la puissance de la différence de pression étant comparée à la valeur limite prédéterminée et, lors du dépassement de la valeur limite, la présence d'une hémorragie paravasculaire en est déduite.

3. Procédé selon la revendication 1 ou 2, **caractérisé en ce que** la valeur de la puissance de la valeur de la différence entre la pression mesurée et la valeur de référence prédéterminée est déterminée, la valeur de la puissance de la valeur de la différence de pression étant comparée à la valeur limite prédéterminée et, lors du dépassement de la valeur limite, la présence d'une hémorragie paravasculaire en est déduite.

4. Procédé selon l'une des revendications 1 à 3, pour la détection d'une hémorragie paravasculaire pendant un traitement extracorporel du sang, dans lequel du sang s'écoule d'un accès vasculaire artériel par l'intermédiaire d'un embranchement artériel d'une circulation sanguine extracorporelle dans un dispositif de traitement de sang et s'écoule du dispositif de traitement de sang par l'intermédiaire d'un embranchement veineux de la circulation extracorporelle vers un accès vasculaire veineux, la pression artérielle étant mesurée dans l'embranchement artériel et/ou la pression veineuse dans l'embranchement veineux de la circulation extracorporelle étant mesurée, **caractérisé en ce que** une première valeur d'une fonction non linéaire de la différence entre la pression artérielle mesurée et une valeur de référence prédéterminée pour la pression artérielle et/ou une deuxième valeur d'une fonction non linéaire de la différence entre la pression veineuse mesurée et une valeur de référence prédéterminée pour la pression veineuse est déterminée, la première valeur étant comparée avec une première valeur limite et/ou la deuxième valeur étant comparée avec une deuxième valeur limite et, lors du dépassement de la première valeur limite respectivement de la deuxième valeur limite, la présence d'une hémorragie paravasculaire en est déduite.

5. Procédé selon la revendication 4, **caractérisé en ce qu'**une première valeur de la puissance de la valeur de la différence entre la pression artérielle mesurée et une valeur de référence prédéterminée pour la pression artérielle et/ou une deuxième valeur de la puissance de la valeur de la différence entre la pression veineuse mesurée et une valeur de référence prédéterminée pour la pression veineuse est déterminée, la première valeur étant comparée avec une première valeur limite et/ou la deuxième valeur étant comparée avec une deuxième valeur limite et, lors du dépassement de la première valeur limite respectivement de la deuxième valeur limite, la présence d'une hémorragie vasculaire en est déduite.

6. Procédé selon la revendication 4, **caractérisé en ce qu'**une première valeur de l'intégrale de la valeur de la différence entre la pression artérielle mesurée et une valeur de référence prédéterminée pour la pression artérielle est déterminée sur un intervalle $t_2$-$t_1$ prédéterminé et la première valeur est comparée à une première valeur limite prédéterminée et, lors du dépassement de la première valeur, la présence d'une hémorragie paravasculaire en est déduite et/ou une deuxième valeur de l'intégrale de la valeur de la différence entre la pression veineuse mesurée et une valeur de référence prédéterminée pour la pression veineuse est déterminée sur un intervalle $t_2$-$t_1$ prédéterminé et la deuxième étant comparée à une deuxième valeur limite et, lors du dépassement de la deuxième valeur limite, la présence d'une hémorragie paravasculaire en est déduite.

7. Procédé selon la revendication 5 ou 6, **caractérisé en ce que** le double, le triple ou le quadruple de la puissance de la valeur de la différence entre la pression artérielle mesurée et une valeur de référence prédéterminée pour la pression artérielle, respectivement le double, le triple ou le quadruple de la puissance de la valeur de la différence entre la pression veineuse mesurée et une valeur de référence prédéterminée pour la pression veineuse est déterminée.

8. Procédé selon l'une des revendications 1 à 7, **caractérisé en ce que**, lorsqu'une hémorragie paravasculaire est détectée, une alarme optique et/ou acoustique est déclenchée.

9. Procédé selon l'une des revendications 1 à 8, **caractérisé en ce que**, lorsqu'une hémorragie paravasculaire est détectée, le flux sanguin est interrompu.

10. Dispositif de détection d'une hémorragie paravasculaire lors d'un apport de sang vers un accès vasculaire par l'intermédiaire d'une conduite et/ou lors d'un prélèvement de sang dans un accès vasculaire par l'intermédiaire d'une conduite, le dispositif comprenant, pour la détection d'une hémorragie paravasculaire, un dispositif de mesure de la pression dans la conduite, **caractérisé en ce que** le dispositif de détection d'une hémorragie paravasculaire comprend une unité d'analyse et de calcul (20) conçue de façon à ce qu'une fonction non linéaire de la différence entre la pression mesurée dans la conduite et une valeur de référence prédéterminée est déterminée, la valeur de la fonction non linéaire est comparée à une valeur limite prédéterminée et lors du dépassement de la valeur limite, la présence d'une hémorragie paravasculaire en est déduite.

**11.** Dispositif selon la revendication 10, **caractérisé en ce que** l'unité d'analyse et de calcul (20) est conçue de façon à ce que la valeur de la puissance de la différence entre la pression mesurée et la valeur de référence prédéterminée est déterminée, la valeur de la puissance de la différence de pression est comparée avec la valeur limite prédéterminée et lors du dépassement de la valeur limite, la présence d'une hémorragie paravasculaire en est déduite.

**12.** Dispositif selon la revendication 10 ou 11, **caractérisé en ce que** l'unité d'analyse et de calcul (20) est conçue de façon à ce que la valeur de la puissance de la valeur de la différence entre la pression mesurée et la valeur de référence prédéterminée est déterminée, la valeur de la puissance de la valeur de la différence de pression est comparée avec la valeur limite prédéterminée et lors du dépassement de la valeur limite, la présence d'une hémorragie paravasculaire en est déduite.

**13.** Dispositif selon l'une des revendications 10 à 12, pour la détection d'une hémorragie paravasculaire pendant un traitement extracorporel du sang avec un dispositif extracorporel de traitement de sang, ce dispositif extracorporel de traitement de sang comprenant :

une conduite de sang artériel d'une circulation sanguine extracorporelle, qui est reliée, à une extrémité, à l'entrée d'un dispositif de traitement de sang et est muni à l'autre extrémité, d'une connexion artérielle pour un accès vasculaire artériel,
une conduite de sang veineux d'une circulation sanguine extracorporelle, qui est reliée, à une extrémité, à la sortie d'un dispositif de traitement de sang et est muni à l'autre extrémité, d'une connexion veineuse pour un accès vasculaire veineux,
et un dispositif de mesure de la pression artérielle dans l'embranchement artériel et/ou de la pression veineuse dans l'embranchement veineux de la circulation sanguine extracorporelle,
**caractérisé en ce que**
le dispositif de détection d'une hémorragie paravasculaire comprend une unité d'analyse et de calcul (20) qui est conçue de façon à ce qu'une première valeur d'une fonction non linéaire de la différence entre la pression artérielle mesurée et une valeur de référence prédéterminée pour la pression artérielle et/ou une deuxième valeur d'une fonction non linéaire de la différence entre la pression veineuse mesurée et une valeur de référence prédéterminée pour la pression veineuse est déterminée, la première valeur étant comparée à une première valeur limite prédéterminée et/ou la deuxième valeur étant comparée avec une deuxième valeur limite prédéterminée et lors du dépassement de la première valeur limite, respectivement de la deuxième valeur limite, la présence d'une hémorragie paravasculaire en est déduite.

**14.** Dispositif selon la revendication 13, **caractérisé en ce que** le dispositif de détection d'une hémorragie paravasculaire comprend une unité d'analyse et de calcul (20) qui est conçue de façon à ce qu'une première valeur de la puissance de la valeur de la différence entre la pression artérielle mesurée et une valeur de référence prédéterminée pour la pression artérielle et/ou une deuxième valeur de la puissance de la valeur de la différence entre la pression veineuse mesurée et une valeur de référence prédéterminée pour la pression veineuse est mesurée, la première valeur étant comparée à une première valeur limite prédéterminée et/ou la deuxième valeur étant comparée avec une deuxième valeur limite prédéterminée et lors du dépassement de la première valeur limite respectivement de la deuxième valeur limite, la présence d'une hémorragie paravasculaire en est déduite.

**15.** Dispositif selon la revendication 13, **caractérisé en ce que** le dispositif de détection d'une hémorragie paravasculaire comprend une unité d'analyse et de calcul (20) qui comprend :

des moyens (20A) pour le calcul d'une première valeur de l'intégrale de la puissance de la valeur de la différence entre la pression artérielle mesurée et une valeur de référence prédéterminée pour la pression artérielle sur un intervalle de temps $t_2 - t_1$ prédéterminé et/ou d'une deuxième valeur de l'intégrale de la puissance de la valeur de la différence entre la pression veineuse mesurée et une valeur de référence prédéterminée pour la pression veineuse sur un intervalle de temps $t_2 - t_1$ prédéterminé et

des moyens (20B) pour la comparaison de la première valeur avec une première valeur limite prédéterminée et/ou de la deuxième valeur avec une deuxième valeur limite prédéterminée.

**16.** Dispositif selon la revendication 14 ou 15, **caractérisé en ce que** l'unité d'analyse et de calcul (20) est conçue de façon à ce que le double, le triple ou le quadruple de la puissance de la valeur de la différence entre la pression artérielle mesurée et une valeur de référence prédéterminée pour la pression artérielle, respectivement le double, le triple ou le quadruple de la puissance de la valeur de la différence entre la pression veineuse mesurée et une valeur de référence prédéterminée pour la pression veineuse est déterminée.

**17.** Dispositif selon l'une des revendications 10 à 16, **caractérisé en ce que** le dispositif de détection d'une hémorragie paravasculaire comprend une unité d'alarme (22) qui, lors de la détection d'une hémorragie paravasculaire, déclenche une alarme optique et/ou acoustique.

**18.** Dispositif selon l'une des revendications 13 à 17, **caractérisé en ce que** le dispositif de détection d'une hémorragie paravasculaire comprend des moyens (20) pour la génération d'un signal de commande pour l'interruption du flux sanguin lors de la détection d'une hémorragie paravasculaire.

**19.** Dispositif selon l'une des revendications 13 à 18, **caractérisé en ce que** le dispositif de détection d'une hémorragie paravasculaire comprend des moyens (20) pour la génération d'un signal de commande pour l'interruption du flux sanguin du dispositif de traitement de sang extracorporel lors de la détection d'une hémorragie paravasculaire.

**20.** Dispositif de traitement de sang extracorporel avec
une conduite de sang artériel (5) d'une circulation sanguine extracorporelle (I), reliée à une extrémité avec l'entrée (3A) d'un dispositif de traitement de sang (1) et munie, à l'autre extrémité, d'un branchement artériel (5A) pour un accès vasculaire artériel,
une conduite de sang veineux (7) d'une circulation sanguine extracorporelle (I), reliée à une extrémité avec la sortie (3B) du dispositif de traitement de sang (1) et munie, à l'autre extrémité, d'un branchement veineux (7A) pour un accès vasculaire veineux,
et un dispositif (18) de mesure de la pression artérielle dans l'embranchement artérielle (5) et/ou de la pression veineuse dans l'embranchement veineux (7) de la circulation sanguine extracorporelle (I),
**caractérisé en ce que** le dispositif de traitement de sang extracorporel comprend un dispositif selon l'une des revendications 13 à 19.

**21.** Dispositif de traitement de sang extracorporel selon la revendication 20, **caractérisé en ce que** le dispositif de traitement de sang extracorporel comprend des moyens (6, 13) permettant d'interrompre le flux sanguin dans la circulation sanguine extracorporelle.

Fig. 1

Fig. 2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102006032815 A1
- EP 0995451 A2
- US 4530696 A
- US 5423743 A
- US 4846792 A
- EP 0817653 B1
- EP 1930035 A1